(19) Europäisches Patentamt
European Patent Office
Office européen des brevets

(11) **EP 0 723 452 B1**

(12) **EUROPEAN PATENT SPECIFICATION**

(45) Date of publication and mention
of the grant of the patent:
**29.12.2004 Bulletin 2004/53**

(51) Int Cl.⁷: **A61K 33/00**, A61K 33/42,
A61K 33/10

(21) Application number: **94930721.9**

(22) Date of filing: **11.10.1994**

(86) International application number:
**PCT/US1994/011548**

(87) International publication number:
**WO 1995/010290 (20.04.1995 Gazette 1995/17)**

(54) **ANTACID PHARMACEUTICAL COMPOSITION**

PHARMAZEUTISCHE ANTACIDZUSAMMENSETZUNG

COMPOSITION PHARMACEUTIQUE ANTIACIDE

(84) Designated Contracting States:
**BE CH DE DK ES FR GB GR IT LI**

(30) Priority: **13.10.1993 US 136570**
**05.10.1994 US 316416**

(43) Date of publication of application:
**31.07.1996 Bulletin 1996/31**

(73) Proprietor: **Warner-Lambert Company LLC**
**Morris Plains, New Jersey 07950 (US)**

(72) Inventors:
• **GEORGIADES, Constantine**
**East Brunswick, NJ 08816 (US)**
• **BUCH, R., Michael**
**Hackettstown, NJ 07840 (US)**
• **ENGELMAN, E., Eric**
**East Stroudsburg, PA 18301 (US)**
• **VOLPE, Frank, A.**
**Kinnelon, NJ 07405 (US)**

(74) Representative: **Mansmann, Ivo et al**
**Warner-Lambert Company,**
**Legal Division,**
**Patent Department,**
**c/o Gödecke AG**
**79090 Freiburg (DE)**

(56) References cited:
DE-A- 1 915 798      GB-A- 922 038
GB-A- 1 056 212      US-A- 3 384 546

## EP 0 723 452 B1

**Description**

Background of the Invention

The present invention relates generally to pharmaceutical compositions comprising active agents useful in the treatment of upper gastrointestinal disorders such as what is commonly referred to as sour stomach, upset stomach, heartburn, gas, acid indigestion. Pharmaceutical compositions containing antacid agents useful for treating gastrointestinal problems are widely used and have been around for quite some time. They vary to some extent in the active ingredients and other excipients and increasingly vary with respect to the other ingredients used that are responsible for flavor, texture, mouthfeel and mechanisms of delivery.

Ingestible pharmaceutical agents capable of relieving gastrointestinal distress typically work by neutralizing stomach acid. Predominantly, these antacids comprise a carbonate or hydroxide of an alkaline earth metal salt such as calcium carbonate or magnesium hydroxide. In terms of simple chemistry, using calcium carbonate as an example, the neutralization reaction occurs as follows

$$1. H^+(acid) + CaCO_3 \rightarrow HCO_3^- + Ca^{++}$$

$$2. H^+ + HCO_3^- \rightarrow H_2O + CO_2\uparrow$$

These agents unfortunately also present a chalky, gritty mouthfeel which must somehow be taste-masked in order to insure good patient compliance. Whereas certain levels of the active agent are needed in order to achieve the optimal buffering profile of pH 3.0-5.0, large amounts of the metal salt in the tablet or liquid dosage form can substantially detract from its palatability. A happy medium is required then, that provides both quick, upfront and sustained relief while at the same time is incorporated in the delivery system at levels that do not render the medication bad tasting.

United States Patent No. 3,621,094 and German Patent No. DE 1 915 798 both to Mayrom et. al. disclose an aqueous pharmaceutical composition for the treatment of acid indigestion and ulcers comprising a high concentration of antacid and a combination of monobasic calcium phosphate and a non-toxic alkali metal or alkaline gluconate salt such as sodium, potassium, calcium. It is asserted that the use of monobasic calcium phosphate and the gluconate salt permits the incorporation of higher levels of antacid than previously possible. These high levels of antacid are particularly necessary in the treatment of peptic ulcers. It is asserted that the composition is effective in reducing gastrointestinal discomfort while at the same time affording the patient a palatable tablet form.

United States Patent No. 4,786,502 to Chapura et. al. discloses palatable pharmaceutical compositions useful in the treatment of gastrointestinal disorders comprising a bismuth or metallic antacid salt such as calcium carbonate or aluminum phosphate, a lipid material with a melting point of from 26°C to 37°C, a hydrophilic dispersant such as a sugar alcohol and an emulsifier. The compositions allegedly allow for the incorporation of sufficiently high amounts of the active for quick and long lasting relief while at the same time providing for a good tasting, smooth textured means of delivery.

United States Patent 3,573,006 to Shik et. al. discloses an antacid composition comprising a polymeric aluminum and magnesium hydroxy complex in a pharmaceutically acceptable carrier for the relief of stomach acid distress, and is allegedly pleasant tasting with a non-gritty mouthfeel.

United States Patent No. 4,495,087 to Wright et. al. teaches a palatable antacid consisting of an aluminum hydroxycarbonate gel that is effective in buffering high levels of stomach acid.

Finally, U.S. Patent No. 4,592,039 to Duvall et. al. discloses an effervescent analgesic antacid with reduced sodium content for those who must restrict their sodium intake. Aspirin, acetaminophen, ibuprofen and the like are combined with an antacid selected from the group consisting of citric acid, sodium bicarbonate, calcium carbonate, potassium bicarbonate and other fillers, flavors and sweeteners.

It is relatively clear then that there have been many formulations in the past developed to reduce gastrointestinal distress that utilize metal salts such as calcium carbonate, dihydroxy aluminum sodium carbonate (DASC), calcium phosphate, sodium citrate, magnesium carbonate, monobasic calcium phosphate aluminum carbonate, aluminum hydroxide and the like. Few of these however, have been able to provide an antacid that is both fast acting for immediate upfront relief, long lasting for sustained relief and yet good tasting with a palatable mouthfeel. Whereas DASC formulations are able to achieve optimal buffering, few have provided an optimal buffering profile of from 3.0 to 5.0 pH both quickly and for long periods of time, yet also taste good when administered. And whereas on the one hand they provide relief by neutralizing the stomach acid, the subsequent evolution of $CO_2$ gas can unfortunately result in another form of discomfort for many.

Summary of the Invention

The present invention is a pharmaceutical composition comprising an antacid that provides both immediate, intermediate, and long lasting relief while being low in sodium, aluminum-free and yet possessing a good taste and mouthfeel when administered. The composition contains calcium carbonate in combination with another buffer such as calcium or magnesium citrate, and/or calcium phosphate, either di- or tribasic and mixtures thereof and thereby may also provide a dietary supplement of calcium while offsetting the prevention of phosphate absorption by providing additional phosphate. In a preferred embodiment, a tripartite formulation of calcium carbonate, calcium citrate and calcium phosphate, either di- or tribasic provides both up-front, immediate acid neutralization that is also a sustained long lasting relief without over-compensating to highly basic pH levels. Low in sodium and aluminum- free, these formulations provide not only a source of calcium but generate little gas by utilizing other non-carbonate buffers. The antacid formulation provides optimal acid neutralizing capability and may also comprise other standard antacid filler and flavor ingredients as desired to prepare either a liquid or solid dosage form.

Brief Description of the Drawings

Figure 1 is a graph depicting the continuous acid challenge profile of calcium carbonate/calcium citrate formulations of the present invention as a function of pH versus time.

Figure 2 is a graph depicting the continuous acid challenge profile of calcium carbonate/calcium phosphate formulations of the present invention as a function of pH versus time.

Figure 3 is a graph depicting the continuous acid challenge profile of calcium carbonate/calcium citrate/tri-calcium phosphate formulations of the present invention as a function of pH versus time.

Figure 4 is a graph depicting the continuous acid challenge profile of calcium carbonate/calcium phosphate/magnesium citrate formulations of the present invention as a function of pH versus time.

Detailed Description of the Invention

Antacids and other similar gastrointestinal medications relieve symptoms such as the burning, painful sensation of heartburn and indigestion through neutralization of acid formed in the stomach. Few antacids are available however, that afford both immediate, upfront relief and sustained, long lasting relief over time. It is well known that the ideally neutralized stomach environment is at a pH of from 3.0 to 5.0, and it is when acid buildup lowers the pH below 3.0 that mucosal erosion, the pain of heartburn, acid indigestion and associated discomfort sets in. Obviously then, a rapid correction and maintenance of the stomach pH in this optimal realm will alleviate most discomfort.

The formulations of the present invention are based upon the theory that the buffering profile of a mix of ingredients with different solubilities will provide acid neutralization action at different times and hence will result in overlapping pH profiles. The ability of a medication to relieve the symptoms associated with heartburn, sour stomach, and acid indigestion is measured in terms of its acid neutralizing capacity (ANC) and is a function of its ability to maintain the stomach at conditions with pH values of from 3.0 to 5.0 over time. The formulations of the present invention were discovered to surprisingly and unexpectedly neutralize strongly acidic conditions to this range immediately upon consumption and for extended periods of time. For example, calcium carbonate ($CaCO_3$) provides substantially long lasting, sustained action relief. Its onset is more delayed and slower than that of the other more soluble components. Calcium citrate provides substantially greater up-front and immediate release so that the two, when combined together provide a broader spectrum of acid neutralizing therapy.

The acid neutralizing capacity for each buffer varies and hence the amount of buffer incorporated in an antacid dosage form is dependent upon the relative ANC's for each component. As used herein, the amount of buffer used in the claimed formulations will be defined in terms of its ANC and the amount contributed by a component as a percentage of the total.

Calcium carbonate has an acid neutralizing capacity of 20 per gram; that is, one (1) gram of calcium carbonate will neutralize 20 (mls.) of 1N acid or 20 meq. thereof. Calcium citrate has an ANC of 6.5 so that 1. 0 gram of calcium citrate will neutralize 6.5 mls. 1N acid or 6.5 meq. thereof. Magnesium citrate has an ANC of 3.5 so that 1.0 gram of magnesium citrate will neutralize 3.5 mls. 1N acid or 3.5 meq. thereof. In one embodiment of the present invention where only a two-component formulation is contemplated, calcium carbonate is incorporated in an amount that will contribute from 20% to 80% of the total ANC while the calcium citrate portion is incorporated in the tablet in an amount that will contribute from 80% to 20% of the total ANC. Preferably, the two will be incorporated in amounts that will each contribute 50% of the total ANC of the dose form.

In another embodiment, calcium carbonate is incorporated in an amount that will contribute from 20 to 80 % of the total ANC while the calcium phosphate portion is incorporated in the tablet in an amount that will contribute from 80 to 20 % of the total ANC.

One preferred embodiment of the present invention is a tripartite system in which calcium carbonate, calcium citrate and calcium phosphate, either di- or tri-basic, are combined in ratios which will achieve the optimal buffering levels both immediately and for sustained periods of time. As before, the amounts of each will comprise a percentage of the total that effectively contributes to the overall ANC of the formulation. The exact amounts in terms of weight percentage will vary depending upon the size of the tablet formulated. The weight amount, however can easily be calculated by one skilled in the art knowing the ratios and the respective ANC's of each component. Preferably, calcium carbonate will be incorporated in an amount that will contribute from 20% to 70% of the total ANC. Calcium citrate will be incorporated in an amount that will contribute from 20% to 35% of the total ANC of the system while the calcium phosphate, di- or tri-basic, is incorporated in an amount that will contribute from about 20% to 50% of the total ANC of the antacid composition. More preferably, each component will be incorporated in an amount that will contribute from 30% to 40% of the total ANC while most preferably each component will be incorporated in an amount that will contribute about one-third of the total ANC of the formulation.

Another preferred embodiment of the present invention is a tripartite system in which calcium carbonate, calcium phosphate, either di- or tri- basic, and magnesium citrate are combined in ratios which will achieve the optimal buffering levels both immediately and for sustained periods of time. As before, the amounts of each will comprise a percentage of the total that effectively contributes to the overall ANC of the formulation. The exact amounts in terms of weight percentage will vary depending upon the size of the tablet formulated. The weight amount, however can easily be calculated by one skilled in the art knowing the ratios and the respective ANC's of each component. Preferably, calcium carbonate will be incorporated in an amount that will contribute from 20% to 80% of the total ANC. Magnesium citrate will be incorporated in an amount that will contribute from 5% to 30% of the total ANC of the system while the calcium phosphate, di- or tri-basic, is incorporated in an amount that will contribute from 15% to 50% of the total ANC of the antacid composition. More preferably, magnesium citrate will be incorporated in an amount that will contribute 8% to 15% of the total ANC, while the remainder of the ANC will be contributed by calcium carbonate and calcium phosphate, di- or tri-basic.

In another preferred embodiment, the tripartite system comprises a combination of calcium carbonate, calcium citrate and calcium phosphate. In these systems calcium carbonate, calcium citrate and calcium phosphate are preferably incorporated into the compositions in amounts that are effective in contributing to the total acid neutralization capacity in ratios of from 60:20:20 to 10:35:45, or preferably in a ratio of 30:30:40 respectively.

Tripartite system comprising calcium carbonate, calcium phosphate and magnesium citrate are also encompassed by the invention. In these systems calcium carbonate, calcium phosphate and magnesium citrate are preferably incorporated into the compositions in amounts that are effective in contributing to the total acid neutralization capacity in a ratio of 45:45:10 respectively.

These formulations then surprisingly and unexpectedly are able to achieve the optimal buffering profile as achieved by dihydroxy aluminum sodium carbonate (DASC) while at the same time providing a source of calcium that is sodium and aluminum free. And despite the presence of this calcium in the system, the additional phosphate that is made available through the calcium phosphate offsets the prevention of phosphate absorption otherwise caused by the calcium. Moreover, the tripartite system allows for an effective acid neutralizing capacity that will produce less gas in the digestive system. The evolution of $CO_2$ gas from the neutralization reaction can naturally cause additional discomfort for obvious reasons, but in the formulations of the present invention, the carbonate level can be reduced with a corresponding increase in the amount of calcium phosphate and calcium citrate so as to provide alternative dose forms for those individuals who are more sensitive to this development. The following Table 1 discloses three tripartite antacid formulations comprising calcium carbonate, calcium citrate, and calcium phosphate. Also disclosed are the relative percent ANC contributions and the weight in milligrams of each component in a standard antacid tablet with a total ANC of 7.5.

TABLE 1

| Acid Neutralization Capacity Percent Contribution cal.carb./cal. cit./cal.phos | Total Calcim Carbonate | Total Calcium Citrate | Total Calcium Phosphate |
|---|---|---|---|
| a) 37.5/25.0/37.5 | 140.0 mg. | 290.0 mg | 210.0 mg. |
| b) 25.0/25.0/50 | 95.0 mg. | 290.0 mg. | 280.0 mg. |

TABLE 1   (continued)

| Acid Neutralization Capacity Percent Contribution cal.carb./cal. cit./cal.phos | Total Calcim Carbonate | Total Calcium Citrate | Total Calcium Phosphate |
|---|---|---|---|
| c) 50.0/25.0/25.0 | 190.0 mg. | 290.0 mg. | 140.0 mg. |

Formulation b for example, will result in the least amount of gas production since the least amount of calcium carbonate is used in terms of an acid neutralizing component.

Table 2 discloses three tripartite antacid formulations comprising calcium carbonate, calcium phosphate and magnesium citrate. Also disclosed are the relative percent ANC contributions and the weight in milligrams of each component in a standard antacid tablet with a total ANC of 10.9.

TABLE 2

| Acid Neutralization Capacity Percent Contribution cal.carb./ cal.phos./ mag. c it. | Total Calcium Carbonate | Total Calcium Phospate | Total Magnesium Citrate |
|---|---|---|---|
| a) 67.9/22.9/9.2 | 368.4 mg. | 182.0 mg | 284.1 mg. |
| b) 45.4/45.4/9.3 | 249.5 mg. | 369.6 mg. | 287.6 mg. |

Formulation b for example, will result in the least amount of gas production since the least amount of calcium carbonate is used in terms of an acid neutralizing component.

Along with the actives set forth above, the antacid formulations of the present invention will include a number of carrier materials, and fillers and other minor excipients normally found in most solid and liquid dosage antacid compositions. Optionally, another active such as simethicone may be added to reduce any discomfort resulting from the formation of gas that may accompany the other digestive disorders and is incorporated in amounts well known in the art. Inactive ingredients, for example, may include a bulking agent such as starch, and starch derivatives, cellulose and cellulose derivatives, corn syrup, sugars, sugar alcohols, lubricants such as magnesium stearate and mineral oil, tableting agents such as polyethylene glycol and titanium dioxide, silica, assorted F.D.& C. dyes, flavors, high intensity sweeteners silicates, talc and mixtures thereof. The exact types and amounts of these ingredients are well known and can be readily determined by one skilled in the art.

The antacid compositions of the present invention can be prepared as chewable tablet dosage forms or as liquid dosage forms.

The antacid formulations of the present invention were effective in providing upfront and long lasting relief of stomach heartburn, acid indigestion and sour stomach through the reduction in stomach acidity to a pH of from about 3.0 to about 5.0. The effectiveness of these formulations was borne out by what is known in the art as the Modified Rosset Rice procedure or the Acid Challenge test whereby the pH of a system treated with a particular formulation is measured over time. Under this procedure, twenty (20) milliequivalents (megs.) of the antacid formulation to be tested are mixed with ten (10) meg. of acid (100 mls. of 0.1M HCl). This is mixed thoroughly and the pH is monitored as an additional one (1) ml. of the 0.1M HCl is added per minute. The measured pH is recorded at regular intervals and plotted as a function of time.

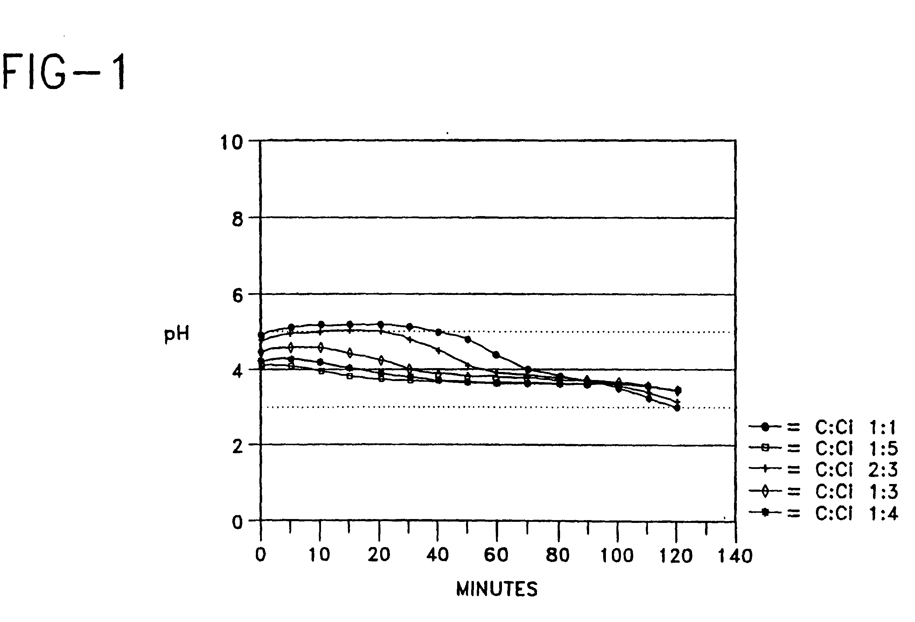

Referring now to the drawings, Fig. 1 depicts the continuous acid challenge profiles of five (5) calcium carbonate/calcium citrate formulations over a 2 hour period. All five formulations maintained pH values in the in-vitro system from about 2.9 to about 5.1. Increasing the citrate to carbonate ratio improves the kinetics of the buffering system, providing the optimal pH (3-5) more rapidly without compromising the duration of neutralization.

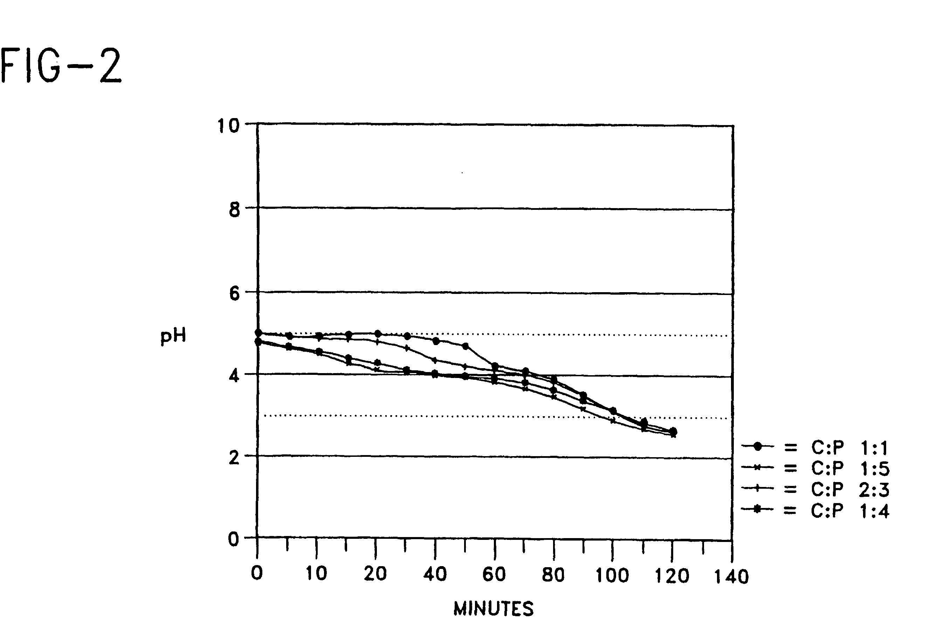

Referring now to Fig. 2, four formulations comprising calcium carbonate/tri-basic calcium phosphate were challenged at various weight ratio amounts. A similar increase in buffering kinetics to that discussed in Figure 1 was observed when the ratio of phosphate to carbonate was increased.

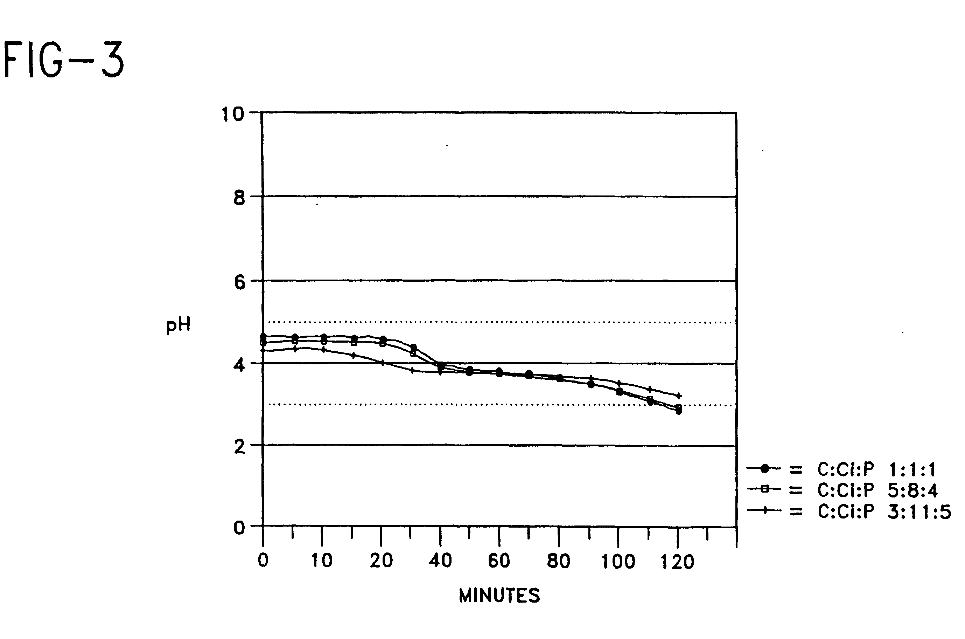

Fig. 3 shows the result for a tripartite formulation comprising calcium carbonate/calcium citrate/tri-basic calcium phosphate in three different ratios. Whereas the optimal pH range of 3. 0 to 5.0 was fairly well established and maintained (i.e. 2.9 to 4.7) all three formula ratios provided substantially similar results.

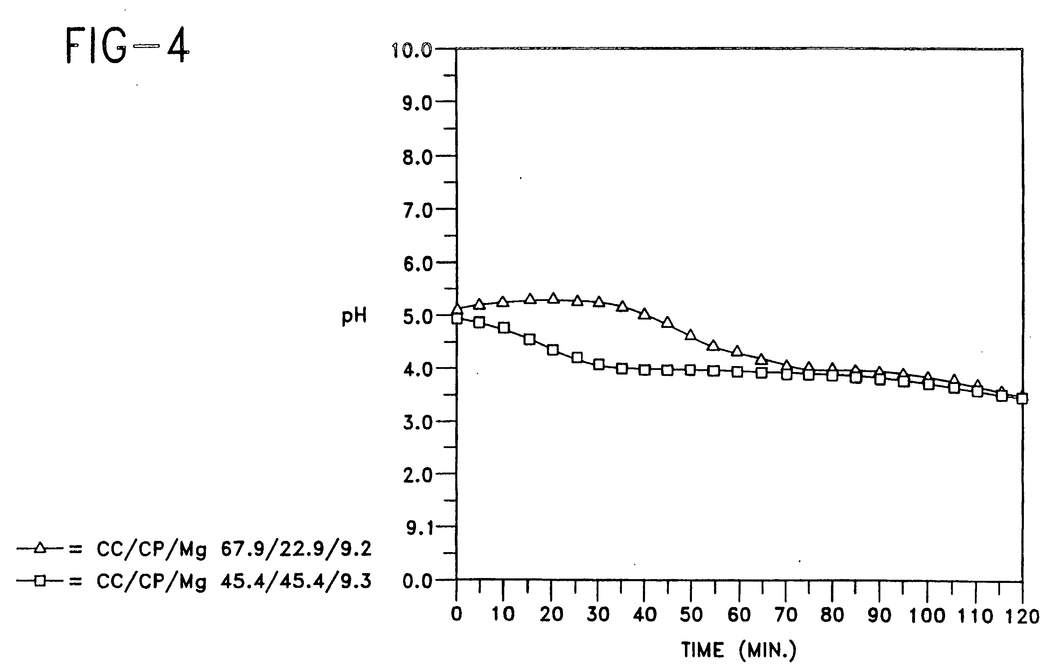

Fig. 4 shows the result for a tripartite formulation comprising calcium carbonate/magnesium citrate/tri-basic calcium phosphate in three different ANC ratios. Whereas the optimal pH range of 3.0 to 5.0 was fairly well established and maintained (i.e. 3.1 to 5.2) both formula ratios provided substantially similar results.

# EP 0 723 452 B1

**Claims**

1. An antacid pharmaceutical composition for the immediate and long lasting relief of gastrointestinal distress and/or dietary calcium supplement comprising a first active buffering agent which is calcium carbonate, and a second and optionally third active buffering agents, inactive carrier material and excipients, wherein said second and/or third active buffering agent is selected from calcium or magnesium citrate, and/or calcium phosphate wherein the phosphate is either di- or tribasic, and mixtures thereof, **characterized in that**

   a. the active ingredients solely comprise calcium carbonate and calcium citrate in amounts that contribute to the total acid neutralization capacity of the composition in ratios of from 20: 80 to 80:20, or

   b. the active agents solely comprise calcium carbonate and calcium phosphate in amounts that are effective in contributing to the total acid neutralization capacity of the composition in ratios of from 20:80 to 80:20, respectively.

2. The antacid composition of claim 1 b) further comprising calcium citrate as a third buffering agent.

3. The antacid composition of claim 1 further comprising magnesium citrate. as a third buffering agent.

4. The antacid composition of claim 2 wherein said calcium carbonate, calcium citrate and calcium phosphate are incorporated into the compositions in amounts that are effective in contributing to the total acid neutralization capacity in ratios of from 60 : 20 : 20 to 10:35:45, respectively.

5. The antacid composition of claim 4 wherein said calcium carbonate, calcium citrate and calcium phosphate are incorporated into the composition in amounts that are effective in contributing to the total acid neutralization capacity of the composition in a ratio of 30:30:40, respectively.

6. The antacid composition of claim 3 wherein said calcium carbonate, calcium phosphate and magnesium citrate are incorporated into the composition in amounts that are effective in contributing to the total acid neutralization capacity of the composition in ratios of from 80:15:5 to 20:50:30, respectively.

7. The antacid composition of claim 6 wherein said calcium carbonate, calcium phosphate and magnesium citrate are incorporated into the composition in amounts that are effective in contributing to the total acid neutralization capacity of the composition in a ratio of 45:45:10.

8. The antacid composition of claim 1 to 7 wherein said carrier materials are selected from cellulose, cellulose derivatives, starches, sugars, sugar alcohols, silicates, corn syrup, silica, mineral oil, polyethylene glycol, talc and mixtures thereof.

9. The antacid composition of claims 1 to 8 wherein said excipients are selected from tabletting agents, lubricating agents, simethicone, artificial high intensity sweeteners, F.D. & C. food colors, flavors and mixtures thereof.

10. The antacid composition of claims 1 to 9 prepared as a chewable tablet dosage form.

11. The antacid composition of claim 1 to 9 prepared as a liquid dosage form.

**Patentansprüche**

1. Eine säurebindende pharmazeutische Zubereitung für die sofortige und lang anhaltende Abhilfe bei Magen/Darm-Leiden und/oder Calcium-Nahrungsergänzung umfassend ein erstes wirksames Pufferagens, das Calciumcarbonat ist und ein zweites und optional ein drittes wirksames Pufferagens, inaktives Trägermaterial und Hilfsstoffe, worin besagtes zweites und drittes wirksames Pufferagens ausgewählt ist aus Calcium- oder Magnesiumcitrat und/oder Calciumphosphat, worin das Phosphat entweder di- oder tri-basisch ist sowie Mischungen hiervon, dadurch charakterisiert, dass

   a. die wirksamen Bestandteile, ausschließlich Calciumcarbonat und Calciumcitrat, in solchen Mengen umfassen, dass sie zur gesamten Säureneutralisationskapazität der Zubereitung in Verhältnissen von 20:80 bis 80:

20 beitragen, oder

b. die wirksamen Bestandteile, ausschließlich Calciumcarbonat und Calciumphosphat, in solchen Mengen umfassen, dass sie wirksam zur gesamten Säureneutralisationskapazität der Zubereitung in Verhältnissen von 20:80 bis 80:20 beitragen.

2. Die säurebindende Zubereitung gemäß Anspruch 1 b) ferner Calciumcitrat als drittes wirksames Pufferagens umfassend.

3. Die säurebindende Zubereitung gemäß Anspruch 1 ferner Magnesiumcitrat als drittes wirksames Pufferagens umfassend.

4. Die säurebindende Zubereitung gemäß Anspruch, 2 worin besagtes Calciumcarbonat, Calciumcitrat und Calciumphosphat in den Zubereitungen in solchen Mengen vereinigt sind, dass sie wirksam zur gesamten Säureneutralisationskapazität der Zubereitung in Verhältnissen von 60:20:20 bis 10:35:45 beitragen.

5. Die säurebindende Zubereitung gemäß Anspruch, 4 worin besagtes Calciumcarbonat, Calciumcitrat und Calciumphosphat in der Zubereitung in solchen Mengen vereinigt sind, dass sie wirksam zur gesamten Säureneutralisationskapazität der Zubereitung im Verhältnis 30:30:40 beitragen.

6. Die säurebindende Zubereitung gemäß Anspruch, 3 worin besagtes Calciumcarbonat, Calciumphosphat und Magnesiumcitrat in den Zubereitungen in solchen Mengen vereinigt sind, dass sie wirksam zur gesamten Säureneutralisationskapazität der Zubereitung in Verhältnissen von 80:15:5 bis 20:50:30 beitragen.

7. Die säurebindende Zubereitung gemäß Anspruch, 6 worin besagtes Calciumcarbonat, Calciumphosphat und Magnesiumcitrat in der Zubereitung in solchen Mengen vereinigt sind, dass sie wirksam zur gesamten Säureneutralisationskapazität der Zubereitung im Verhältnis 45:45:10 beitragen.

8. Die säurebindende Zubereitung gemäß der Ansprüche 1-7, worin besagte Trägermaterialien ausgewählt sind aus Cellulose, Cellulosederivaten, Stärken, Zucker, Zuckeralkoholen, Silikaten, Maissirup, Siliziumdioxid, Mineralöl, Polyethylenglykol, Talkum und Mischungen hiervon.

9. Die säurebindende Zubereitung gemäß der Ansprüche 1-8, worin besagte Hilfsstoffe ausgewählt sind aus Tablettierungsstoffen, Gleitmitteln, Simethicon, künstlichen Süssstoffen starker Intensität, F.D. & C. Lebensmittelfarbstoffe, Aromastoffe und Mischungen hiervon.

10. Die säurebindende Zubereitung gemäß der Ansprüche 1-9 hergestellt in Form einer dosierten Kautablette.

11. Die säurebindende Zubereitung gemäß der Ansprüche 1-9 hergestellt in Form einer flüssigen Dosisform.

**Revendications**

1. Composition pharmaceutique antiacide destinée au soulagement immédiat et prolongé des douleurs gastro-intestinales et/ou complément alimentaire à base de calcium comprenant un premier tampon actif qui est du carbonate de calcium, et un second tampon actif, et éventuellement un troisième tampon actif, un matériau de support et des excipients inactifs, dans lequel ledit second tampon actif et/ou ledit troisième tampon actif sont choisis parmi le citrate de calcium ou le citrate de magnésium, et/ou le phosphate de calcium, dans lesquels le phosphate est dibasique ou tribasique, et des mélanges ce ces composés, **caractérisé en ce que**

(a) les ingrédients actifs comprennent uniquement du carbonate de calcium et du citrate de calcium en quantités qui contribuent à la capacité de la composition à neutraliser totalement l'acide, dans des proportions comprises entre 20:80 et 80:20, ou

(b) les agents actifs comprennent uniquement du carbonate de calcium et du phosphate de calcium en quantités qui sont efficaces pour contribuer à la capacité de la composition à neutraliser totalement l'acide, dans des proportions comprises entre 20:80 et 80:20, respectivement.

2. Composition antiacide selon la revendication 1 (b), comprenant en plus du citrate de calcium à titre de troisième tampon.

**EP 0 723 452 B1**

**3.** Composition antiacide selon la revendication 1, comprenant en plus du citrate de magnésium à titre de troisième tampon.

**4.** Composition antiacide selon la revendication 2, dans laquelle lesdits carbonate de calcium, citrate de calcium et phosphate de calcium sont incorporés dans la composition dans des quantités qui sont efficaces pour contribuer à la capacité à neutraliser totalement l'acide, dans des proportions comprises entre 60:20:20 et 10:35:45, respectivement

**5.** Composition antiacide selon la revendication 4, dans laquelle lesdits carbonate de calcium, citrate de calcium et phosphate de calcium sont incorporés dans la composition dans des quantités qui sont efficaces pour contribuer à la capacité à neutraliser totalement l'acide, dans des proportions de 30:30:40, respectivement.

**6.** Composition antiacide selon la revendication 3, dans laquelle lesdits carbonate de calcium, phosphate de calcium et citrate de magnésium sont incorporés dans la composition dans des quantités qui sont efficaces pour contribuer à la capacité à neutraliser totalement l'acide, dans des proportions comprises entre 80:15:5 et 20:50:30, respectivement.

**7.** Composition antiacide selon la revendication 6, dans laquelle lesdits carbonate de calcium, phosphate de calcium et citrate de magnésium sont incorporés dans la composition dans des quantités qui sont efficaces pour contribuer à la capacité à neutraliser totalement l'acide dans des proportions de 45:45:10.

**8.** Composition antiacide selon les revendications 1 à 7, dans laquelle lesdits matériaux de support sont choisis parmi la cellulose, des dérivés de cellulose, des amidons, des sucres, des alcools de sucre, des silicates, du sirop de maïs, la silice, une huile minérale, le polyéthylène glycol, le talc et des mélanges de ces produits.

**9.** Composition antiacide selon les revendications 1 à 8, dans laquelle lesdits excipients sont choisis parmi des agents de pastillage, des agents lubrifiants, la siméthicone, des édulcorants artificiels de forte intensité, des colorants alimentaires F.D. & C., des arômes et des mélanges de ces produits.

**10.** Composition antiacide selon les revendications 1 à 9, préparée sous une forme posologique de comprimé à sucer.

**11.** Composition antiacide selon les revendications 1 à 9, préparée sous une forme posologique liquide.

FIG-1

EP 0 723 452 B1

FIG-2

EP 0 723 452 B1

EP 0 723 452 B1

# FIG-3

pH

MINUTES

- = C:Cl:P 1:1:1
- = C:Cl:P 5:8:4
- = C:Cl:P 3:11:5

FIG—4

EP 0 723 452 B1

12

pH

—△— = CC/CP/Mg  67.9/22.9/9.2
—□— = CC/CP/Mg  45.4/45.4/9.3

TIME (MIN.)